(19) **Europäisches Patentamt**
**European Patent Office**
**Office européen des brevets**

(11) **EP 4 763 074 A1**

(12) **EUROPEAN PATENT APPLICATION**

(43) Date of publication:
**24.06.2026 Bulletin 2026/26**

(21) Application number: **24222789.0**

(22) Date of filing: **23.12.2024**

(51) International Patent Classification (IPC):
**A61B 5/08** (2006.01) **A61B 5/22** (2006.01)
**A61B 5/00** (2006.01) A61B 5/113 (2006.01)
A63B 24/00 (2006.01)

(52) Cooperative Patent Classification (CPC):
**A61B 5/0816; A61B 5/222; A61B 5/4866;**
**A61B 5/7267;** A61B 5/1135; A61B 5/329;
A61B 2503/10; A63B 24/0062

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Charité - Universitätsmedizin Berlin**
**Körperschaft des öffentlichen Rechts**
**10117 Berlin (DE)**

(72) Inventors:
• **Patzak, Andreas**
  **15831 Blankenfelde-Mahlow (DE)**
• **Heinz, Viktor**
  **10587 Berlin (DE)**
• **Bothe, Tomas-Lucca**
  **14050 Berlin (DE)**
• **Anosov, Oleg**
  **14482 Berlin (DE)**

(74) Representative: **Schulz Junghans**
**Patentanwälte PartGmbB**
**Großbeerenstraße 71**
**10963 Berlin (DE)**

(54) **DETERMINATION OF AN INDIVIDUAL ANAEROBIC THRESHOLD**

(57)  The invention relates to a method for determining an individual anaerobic threshold (61), the method comprising the steps of:

a) Acquiring an input signal (10), comprising a respiratory activity signal of a subject (1) executing a physical exercise,

b) Processing said input signal (10), comprising

• A first processing stage (S1) during which an output signal (11) is determined (300) from the input signal (10), wherein the output signal (11) is indicative of a respiration and/or of a property of the respiration of the subject (1),

• A second processing stage (S2) during which the individual anaerobic threshold (61) is determined from the output signal (11), the individual anaerobic threshold (61) being a time point during the physical exercise, or an associated value at this time point, characterized in that

the processing of the input signal (10) comprises submitting (200) the input signal (10) to a machine learning module (20),

wherein the machine learning module (20) comprises one or more trained neural networks (21), that execute the first and/or at least parts of the second processing stage (S1, S2).

Fig. 1

**Description**

The invention relates to a computer-implemented method, a computer program as well as a system for determining an individual anaerobic threshold of a subject.

In the art, determining the individual anaerobic threshold typically involves determining a lactate level in the blood of a subject that is exposed to a stepwise increasing physical load exercise.

In addition or alternatively, it is possible to analyze the composition of respiratory gases from the subject (spiroergometry).

Several characteristic individual parameters are of particular interest, among which are the ventilatory thresholds, VT1 and VT2.

During a cardiopulmonary exercise test (CPET), $VO_2$ and $VCO_2$ may be determined from the respiratory gases, but also the ventilatory thresholds (VT1, VT2) and the respiratory exchange ratio (RER).

VT1 represents the point at which efficient aerobic energy production transitions into a mixed energy production mode, combining aerobic and anaerobic processes. VT2, on the other hand, marks the threshold where energy production predominantly shifts to anaerobic metabolism, defining the individual's sustainable performance limit.

The determination of these parameters typically requires a laboratory setting and controlled exercise conditions, which is considered a disadvantage.

Also, for determining the lactate level in the blood, the subject needs to provide blood samples that are subsequently analyzed.

Precision of the determined lactate level may be influenced by the diet of the subject, the site of blood collection and the technique applied for blood collection. Spiroergometry in turn requires a complex and elaborate experimental setup.

In the framework of professional sports, this may not be a major problem, however, for all athletes below the fully professional level and especially for clinical patients, this limitation represents a significant obstacle to optimized care.

Previous applications, such as those integrated into the smartwatches of well-known manufacturers or dedicated fitness products, estimate the target training zones using approximate formulas (e.g. heart rate 200 beats per minute minus the age of the subject) and/or biometric data. The actual training status of the individual is not considered, and the considerable biological variability is ignored. The benefit of such applications is therefore critically discussed and is at best considered to be very limited.

It is an object of the present invention to provide a method that does not require elaborate experimental settings, that is robust and allows for a precise determination of the individual anaerobic threshold.

The object is achieved by the computer-implemented method having the features of claim 1.

Advantageous embodiments are described in the dependent claims.

According to a first aspect of the invention, a method, particularly a computer implemented-method for determining an individual anaerobic threshold, IAT, comprises the steps of:

- Acquiring an input signal, comprising or consisting of a respiratory activity signal, of a subject, such as a person or an animal executing a physical exercise,
- Processing said input signal, comprising:

  • A first processing stage during which an output signal is determined from the input signal, wherein the output signal is representative of the respiration and/or of a property of the respiration of the subject,
  • A second processing stage during which the individual anaerobic threshold is determined from the output signal, the individual anaerobic threshold being a time point comprised in the input signal during the physical exercise, or an associated value that may be obtained from a synchronized signal at this time point, characterized in that

the processing of the input signal comprises submitting the input signal to a trained machine learning module, wherein the trained machine learning module comprises one or more trained neural networks, that execute the first and/or at least parts of the second processing stage.

The IAT according to the invention can be determined without the use of spiroergometry.

The IAT determined according to the invention essentially corresponds to the spirometric VT2 and the associated Respiratory Exchange Ratio (RER ($1 \pm 0.15$).

Depending on the quality of the input signal acquisition, the precision of IAT determination-comparable to the VT2 in spirometry-ranges from excellent (95%) to moderate (80%).

Additionally, the method according to the invention allows identifying of an onset of a stable metabolic phase, which is referred to as characteristic deflection point in the description, where aerobic and anaerobic energy production are balanced, and lactate accumulation and clearance occur in equilibrium.

The invention allows for extracting the IAT from the respiratory activity signal.

The term "respiratory activity signal" particularly refers to a biological signal which is, in its entirety or in one or multiple of its (sub-)components, correlated, modulated, affected, amplified or suppressed by the respiratory process of the subject.

The respiratory process in particular refers to the breathing of the subject on the macroscopic level in limitation to the cellular level.

The respiratory activity signal comprises one or more components indicative of the respiration.

For example, the respiratory activity signal may comprise or consist of an ECG signal or a pulse signal, that may not primarily intend for the purpose of monitoring or recording respiration.

However, the respiratory activity signal may also be acquired by a chest strap measurement system configured to record respiration.

In particular, the respiratory activity signal is a signal that is not used in the art for determining the IAT.

In the first processing stage, the method according to the invention in essence processes the input signal to arrive at an output signal representing a respiratory contribution in the input signal and/or a property of the said respiratory contribution in the input signal.

In some embodiments, the output signal corresponds to or at least resembles the signal as acquired with spiroergometry, i.e. a respiratory signal.

The term "output signal" may not be limited in interpretation in a way that the output signal is expressly put out on an interface, but the output signal may be solely an intermediate result (e.g. in form of data) that is processed further during the second processing stage.

To render the processing of the first stage robust and efficient, the method employs a machine learning module comprising a trained artificial neural network that is trained to filter the input signal to generate the output signal.

Particularly, the output signal is devoid of pulse or heart features, but may comprise solely information on the respiration and/or one or more properties of respiration.

Exemplary training and network architectures are disclosed in a later section of the present specification.

The inventors found that surprisingly, many biological signals, if not all signals recorded on a subject, comprise information on the respiration of the subject. The inventors further realized that it is possible to filter these signals, termed input signals in the context of the specification, to arrive at the output signal, and "stripping" the input signal from noise and contributions not related to the respiration of the subject. This in turn allows using a wide-variety of input signals for determining the IAT, and not solely signals obtained from spiroergometry.

In particular, the input signal may comprise a thorax motion signal, indicative of the motion or changing of an electrical impedance of the thorax. The electrical impedance is related to a respiratory activity and thus comprised by the term respiratory activity signal. Measuring the thorax motion or impedance may be facilitated by various sensors, e.g. including optical sensors and/or electrical sensors.

In particular, the respiratory activity signal does not stem from a spiroergometry measurement.

In particular, the respiratory activity signal is to be understood as a signal comprising contributions of a respiratory activity and another non-respiratory activity, such as a motion or an impedance of a body part, an endogenous electrical signal, such as an ECG, EEG, or EMG signal, wherein said other activity contributes more than 50%, particularly more than 80% to the respiratory activity signal, particularly more than 90% of a signal strength of the respiratory activity signal.

Particularly, the respiratory activity signal is a mixture of a signal that is associated to the respiratory activity and a signal that is associated to a non-respiratory activity. The signal representing the no-respiratory activity may on average make up for more than 50% of the respiratory activity signal, particularly more than 80% of the respiratory activity signal, particularly more than 90% of a signal strength of the respiratory activity signal.

In the context of the current specification, the term "acquiring" particularly refers to a process of obtaining, generating and/or recording the input signal. Depending on the implementation of the invention, the input signal may be received or read from a non-transitory data storage. Alternatively, and thus optionally, the term "acquiring" may be understood as recording the input signal, which typically involves some kind of sensor.

However, particularly in case the method is executed by a processor or a computer only, the term "acquiring" may be understood as the process of obtaining the input signal, e.g. in form of data from some memory or data storage. That is, the input signal may have been recorded by a sensor and has been stored on a computer-readable temporary or permanent data memory.

Similarly, the IAT may be put out on an interface for recognition by a person or another system. Such an interface may comprise a screen, a display, a loudspeaker, or an electronic other data interface.

The first processing stage is dedicated to the generation of the output signal. The second processing stage is dedicated to the further processing of the output signal to determine the IAT from the output signal.

According to the invention, at least the first processing stage is executed by the machine learning module and, in particular, by one or more artificial neural network(s).

These artificial neural networks can comprise but are not limited to sequential neural networks such as Recurrent Neural Networks, e.g., Long-Short-Term-Memory networks, or convolutional neural networks with one or more dimension or Transformer Networks or Sequence to Sequence Models or WaveNet architectures or Hidden Markov Models or Autoregressive Models or Graph Neural Networks or Echo State Networks or Dynamic Bayesan Networks or derivations or adaptations of all architectures above or general machine-based learning algorithms can be used.

As the output signal is generally considered to be a temporal signal, the IAT is considered a time point in the signal. Obviously, it is possible to synchronously monitor, acquire or record another parameter over time and relate the IAT to the parameter value according to the time of the IAT. That is, the IAT may be understood as the time point at which the respiration exhibits a certain characteristic, or at which a synchronized signal adopts an associated value.

The physical exercise may be configured to increase a load on the exercising subject over time.

The term "processing stage" in the context of the current specification is used for distinguishing specific method steps. It is possible that the outcome of the first processing stage, i.e. the determination of the output signal, may not be explicitly put out but may be generated only as an intermediate computational step that is used for further processing.

In particular, in case the neural network is designed to execute at least a part of the second processing stage, the output signal may not take the form of an output vector of said network, but may be generated only as an intermediate vector between two layers of the network.

In contrast to the existing methods, the processing of the input signal through a neural network to arrive at the output signal allows for various signal sources that provide the input signal, wherein the input signal may stem from different signal sources for different embodiments.

Essential for any embodiment is the use of the neural network that facilitates removal of interfering signal contributions in the input signal, that improves the signal-to-noise ratio, SNR, and that can adapt to different input signal qualities.

According to another embodiment of the invention, the output signal is one or more comprised by the list consisting of:

a) a respiratory signal representing the respiration of the subject,

b) a phase signal of a respiratory signal representing the respiration of the subject, wherein the phase signal represents an instantaneous phase of the respiratory signal,

c) a phase deflection signal representative of a deviation of an instantaneous phase of the respiratory signal from a linear phase of the respiratory signal,

d) an inspiration signal representing time points of maximum inspiration during respiration of the subject,

e) an expiration signal representing time points of maximum expiration during respiration of the subject,

f) a respiration signal representing multiple time points along each respiration cycle in the respiratory signal.

For example, in case the output signal is the respiratory signal representing the respiration of the subject, the signal ideally may correspond very closely or may be even identical to a signal that would have been obtained for example by spiroergometry. It can thus be a quasi-cyclic signal with varying amplitude and frequencies.

A property of the output signal may, therefore, be a property of the respiratory signal. A property of the respiratory signal may for example be a breathing rate (bpm) and/or a breathing amplitude, i.e. an amplitude of the respiratory signal.

Similarly, it is possible, e.g. by way of a Hilbert-Transform of the respiratory signal, to determine an instantaneous phase of the respiratory signal; another property of the respiratory signal. From the Hilbert Transform, the phase signal may be obtained. However, there may be other ways to obtain said phase signal and thus the instantaneous phase.

The Hilbert transform allows investigation of the instantaneous phase and frequency for non-stationary time series.

The resulting phase signal may be approximated by a linear phase function and a non-linear contribution. The phase deflection signal indicates the deviation, e.g. by way of a difference between the linear phase function and the phase signal, of the phase signal from the linear phase signal.

Due to its generic temporal course for a subject exposed to increasing physical loads, the phase deflection signal is a robust function for determining the IAT, as the phase deflection signal comprises obvious features at the IAT.

Other features of the respiratory cycle may be understood as a property of the respiratory signal, and thus may be used as the output signal, such as points of maximum inspiration and/or expiration. Further, other characteristic points in the respiration cycle may be used to make up the output signal.

With regard to f), for example, the respiration can be represented as an envelope curve from the sizes of the R-peaks (or any other peak or characteristic point of the QRS-complex) of an ECG. This results in different numbers of points at any point in the respiratory cycle. The number depends on the ratio of the respiratory rate to the heart rate. At rest, there are approx. 4 heartbeats per respiratory action. The heart rate is also respiration-modulated and accelerates during inspiration and slows down during expiration. This respiratory-synchronous rhythm can also be extracted again (respiratory sinus arrhythmia) and here too there are only as many "sampling points" as there are heartbeats in the respiratory cycle.

According to another embodiment of the invention, the machine learning module consists of one trained neural network that executes the first and at least parts of the second processing stage, particularly the complete second processing stage to obtain the individual anaerobic threshold from the input signal.

This embodiment allows for an end-to-end processing pipeline of the input signal to arrive at the IAT. Further, the integration of processing steps comprised in the first and the second processing stage into a single neural network allows for a simplified computational architecture.

In another embodiment, the one neural network executes the complete first and second processing stage such that the input comprises the input signal and the output of the neural network comprises the determined IAT.

According to another embodiment of the invention, the machine learning module comprises a first trained neural network that executes the first processing stage.

This embodiment allows executing the essential processing of the input signal to obtain the output signal with the neural network. As elaborated in previous paragraphs, the processing with a neural network provides the method with an increased robustness and flexibility.

Processing steps comprised in the second processing stage may be executed by another neural network or by way of non-trained analytical operations.

According to another embodiment of the invention, the machine learning module comprises a second trained neural network that executes at least a part of the second processing stage or the complete second processing stage.

This embodiment allows for an increased robustness and speed as compared to for example analytical operations, such as curve-fitting and / or linear regression. According to another embodiment of the invention, the first and the second neural network are configured to execute all steps of the first and the second processing stage.

This embodiment allows for a robust processing but flexible processing pipeline, that combines the advantages of the previous embodiments relating to two neural networks.

That is, the input of the first neural network is the input signal, the output of the first neural network serves as the input of the second neural network, and the output of the second neural network comprises the IAT.

According to another embodiment of the invention, from the output signal, in case it is not the phase deflection signal, the phase deflection signal is determined, wherein from the phase deflection signal the individual anaerobic threshold is determined. The determination of the phase deflection signal may be done in the first or the second processing stage. As this step may comprise applying a Hilbert Transform to the respiration signal, it may be advantageous to locate this step in the second processing stage, where potentially analytical non-trained operations are performed. As elaborated in a previous paragraph of the current specification, the phase deflection signal is an advantageous representation of the respiration signal to determine the IAT.

According to another embodiment of the invention, determining the anaerobic threshold comprises determining a first and a second linearized region in the phase deflection signal, wherein the first linearized region temporally precedes the second linearized region and is characterized by a - in particular constant - slope with a first slope value, wherein the second linearized region is located adjacent to the first linearized region and exhibits a positive - in particular constant - slope with a second slope value, wherein the second slope value is greater than the first slope value, wherein the anaerobic threshold is assigned to a location of an interface of the second and the third region.

This location of the interface corresponds to a time point in the phase deflection signal, or to the associated value of this time point.

The first and the second linearized region may be determined by way of determining a polygonal chain comprising a first linear segment and a second linear segment and a first connecting vertex connecting the two linear segments that fit the phase deflection signal. The location of the first connecting vertex corresponds to the IAT.

There may be other ways to determine the IAT from the phase deflection signal.

According to another embodiment of the invention, a load signal indicative of a physical load is recorded, wherein an individual anaerobic load value is determined during the second processing stage, wherein the individual anaerobic load value corresponds to the physical load at the individual anaerobic threshold.

The load signal may be recorded synchronously to the input signal or synchronously to the output signal, or it may be synchronized to the input signal or the output signal after recording.

This embodiment allows determining a load value at which the subject reaches the IAT. This embodiment therefore provides a translation of the IAT to an individual load value that may be easier to access and relate to during physical exercise.

The physical load may be stepwise or continuously increased while the subject exercises; each time the load is increased, the load signal increases correspondingly. According to another embodiment of the invention, a heart rate signal indicative of an instantaneous heart rate is acquired, wherein an individual anaerobic heart rate value is determined from the heart rate signal during the second processing stage, wherein the individual anaerobic heart rate value corresponds to the instantaneous heart rate at the position of the individual anaerobic threshold, and/or wherein an individual anaerobic heart rate variability value is determined from the heart rate signal during the second processing stage, wherein the individual anaerobic heart rate variability value corresponds to instantaneous heart rate variability at the position of the individual anaerobic threshold.

The heart rate signal may be recorded synchronously to the input signal or the output signal, or it may be synchronized to the input signal or the output signal after recording.

In some embodiments, the heart rate signal may be comprised in the input signal already, for example in case the input signal is an ECG signal or a pulse signal. That is, the heart rate signal may be the input signal.

Both these values, the individual heart rate value and the individual heart rate variability value, may be used in later training of the subject to monitor the IAT and to have a measure of performance other than the IAT.

In turn, it is possible to determine, whether a person has reached his or her IAT by monitoring the heart rate or the heart rate variability during a subsequent workout. Particularly, the expression "hear rate" refers to an instantaneous heart rate, e.g. reflecting the heart rate on a beat-to-beat basis.

According to another embodiment of the invention, wherein a characteristic deflection point is determined from the phase deflection signal during the second processing stage, wherein the characteristic deflection point is assigned to a location at a change of a slope from negative to positive of the phase deflection signal.

The characteristic deflection point may be used to determine the change of the metabolism from an essentially resting metabolism, to a mainly aerobic metabolic regime under load. It indicates the end of a warming-phase of the exercise. Reaching the characteristic deflection point may be indicated to the subject or other personal that may indicate to adjust the exercise or to increase a load.

According to another embodiment of the invention, determining the characteristic deflection point comprises determining a third linearized region in the phase deflection signal, wherein the third linearized region temporally precedes and is adjacent to the first linearized region and is characterized by a negative slope with a third slope value, wherein the characteristic deflection point is located at an interface of the third and the first region.

This location of the interface corresponds to a time point in the phase deflection signal, or to the associated value of this time point.

The first, second and third linearized region may be determined by way of determining a polygonal chain fitting to the phase deflection signal. The polygonal chain may comprise a first linear segment, a second linear segment, and a third linear segment, wherein a first connecting vertex connects the first and the second linear segments and wherein a second connecting vertex connects the third and the first linear segments. The location of the first connecting vertex corresponds to the IAT, wherein the location of the second connecting vertex corresponds to the characteristic deflection point.

In a diagram of the deflection signal with an ascending time axis pointing from left to right, the third linearized region precedes the first and the second linearized region, i.e. in the diagram it is further left than the first and second linearized regions; similarly, the second connecting vertex precedes the first connecting vertex, i.e. in the diagram it is further left of the first connecting vertex.

Fitting of the polygonal chain may be performed with fitting algorithms such as least-square fitting or the like.

Alternatively, the neural network may be trained to identify the locations of the IAT and the characteristic deflection point.

Similar to the determination of associated values for the IAT, such as the load value or the heart rate value, it is possible to determine an associated load value, a heart rate value and a heart rate variability value for the characteristic deflection point. This is disclosed and elaborated in the following embodiments.

According to another embodiment of the invention, the characteristic deflection point may be determined by a machine learning method that is trained to detect the deflection point. Training may be performed by way of annotated deflection signals. According to another embodiment of the invention, determining the anaerobic threshold comprises matching the phase deflection signal with a superposition of at least two, in particular exactly two, hyperbolic tangent functions, wherein the anaerobic threshold is assigned to a deflection point of the superimposed hyperbolic tangent functions, particularly wherein the superimposed hyperbolic tangent functions exhibit two deflection points at which a curvature of the superimposed hyperbolic tangent functions exhibit a local maximum, particularly wherein the IAT is assigned to the deflection point that occurs for greater times of the deflection signal, i.e. the second deflection point.

Particularly, the characteristic deflection point is assigned to a first local maximum of the superimposed hyperbolic tangent functions that is located before a second local maximum that is assigned to the IAT.

According to another embodiment of the invention, an individual load value associated to the characteristic deflection point is determined during the second processing stage, wherein the individual load value corresponds to the exercise load at the characteristic deflection point.

According to this embodiment, the individual load value corresponds to the time point of the characteristic deflection point in the load signal that may be recorded synchronously to the input signal or synchronously to the respiratory signal, or that may be synchronized to the input signal or the respiratory signal after recording.

This embodiment allows determining a load value at which the subject reaches a stable metabolic phase. This embodiment therefore provides a translation of the characteristic deflection point to a load value that may be easier to access and relate to during physical exercise.

The physical load may be stepwise increased while the subject exercises; each time the load is increased, the load signal is increased correspondingly.

According to another embodiment of the invention, an individual heart rate value associated to the characteristic deflection point is determined during the second processing stage from the heart rate signal, wherein the individual heart rate value corresponds to the heart rate at the associated to the characteristic deflection point, and/or wherein an individual heart rate variability value associated to the characteristic deflection point is determined from the heart rate signal, wherein the individual heart rate variability value corresponds to the heart rate variability at the characteristic deflection point.

The heart rate signal may be recorded synchronously to the input signal or respiratory signal, or it may be synchronized to the input signal or the respiratory signal after recording.

In some embodiments, the heart rate signal may be comprised in the input signal already, for example in case the input signal is an ECG signal or a pulse signal.

Both these values, the individual heart rate value and the individual heart rate variability value, may be used in later training of the subject to monitor the characteristic deflection point and to have a measure of performance other than the characteristic deflection point.

In turn, it is possible to determine whether a person has reached his or her characteristic deflection point by monitoring the heart rate or the heart rate variability during a subsequent workout.

According to another embodiment of the invention, the input signal is selected from one or more of the group consisting of:

> a) An ECG signal,
>
> b) A heart rate signal, particularly recorded with a wearable sport-tracker,
>
> c) An electrical signal comprising the heart rhythm, such as an impedance cardiography signal,
>
> d) A breathing signal,
>
> e) A thorax excursion signal,
>
> f) A pulse wave signal indicative of a pulse of the subject,

The ECG signal may be acquired from a single lead or a from a multi-lead recording.

The ECG signal may be sampled with a sampling frequency of more than 100 Hz.

A wearable sport-tracker may be considered any strap-associated sensors configured to measure a blood flow signal, a heart signal, and/or a pulse of the subject.

The sensor may be wearable by the person by some attachment device or a specific shape of the sensor. The sensor may be comprised by a consumer article, such as a sport watch, a chest strap, a pulse sensor, or in professional medical equipment such as an ECG-system, an X-ray device for monitoring a lung volume. Further, the sensor may be comprised by a camera that is configured to generate image data suitable for extracting the respiratory activity signal.

The sensor signal may eventually be converted to an electrical or electronic signal and used as the input signal.

In the context of the current specification, a breathing signal, may be understood as a signal that is correlated to the respiratory cycle, but may not be considered a respiratory signal.

A thorax excursion signal may be recorded from a movement of the thorax of the subject during exercise. The thorax excursion signal comprises a respiratory component but also components stemming from the heart mechanics, so that the thorax excursion signal requires processing to arrive at the respiratory signal. The thorax excursion signal may be monitored by a camera or by a sensor attached to the subject.

According to this embodiment, the input signal may also be a pulse wave signal indicative of a pulse of the subject.

The pulse may be a volume pulse and/or pressure pulse. The pulse wave signal may be acquired by a pulse oximeter, a chest strap with a pulse sensor, and/or a sport watch, as elaborated in a previous paragraph.

According to another embodiment of the invention, the input signal may be acquired by an ECG system, an EEG system, an EMG-system.

The input signal may be acquired by a thorax- or body-impedance device, configured to measure an impedance of the subject's body or thorax.

According to another embodiment of the invention, the input signal may be acquired via an actigraphy method, a ballistocardiography method, a ballistometry method (serial weight measurement), a pulse plethysmography method.

The input signal may be a respiratory flow signal.

The input may be acquired by way of a respiratory gas analysis, a continuous blood pressure measurement, by way of a mechanical volume signal or a pressure pulse signal. Similarly, the input signal may be acquired by volumetry, e.g., X-ray, ultrasound, camera, thermal imaging, etc...

The input signal may be obtained from a peripheral instantaneous temperature change indicative of skin perfusion.

The signals from the list and the above-mentioned embodiments may not be understood as a respiratory signal, but the method according to the invention allows generating the respiratory signal from these signals, by processing the input signal with one or more neural networks.

According to another embodiment of the invention, after the individual anaerobic heart rate value and/or individual anaerobic heart rate variability value has been determined, a physical training method is executed, particularly repeatedly over a plurality of days, wherein the physical training method comprises determining the heart rate signal with a sensor such as wearable sport-tracker configured to determine a pulse or heart rate signal, wherein the physical training method adjusts an exercise load such that the exercising subject's heart rate is within a range with respect to the anaerobic heart rate value and/or the individual anaerobic heart rate variability value, or wherein the physical training method instructs the exercising subject to adjust a workout intensity such that the heart rate is within the range, particularly wherein said range is -20% to 20%, more particularly wherein said range is -10% to 10% of the anaerobic heart rate value and/or the individual anaerobic heart rate variability value.

Alternatively, the range may be -20% to -10% below or 5% to 20% above the anaerobic heart rate value and/or the individual anaerobic heart rate variability value. This embodiment takes full advantage of the simplicity of the method according to the invention, as it allows monitoring the IAT and therefore for generating real-time feedback regarding an adjustment of the workout intensity.

There are several advantages to training in specific ranges around the IAT. Depending on where the range is set, basic endurance, regeneration and especially return-to-sports training may be executed more efficient. It allows improved rehabilitation after injuries, when training may be executed below the IAT or above the IAT for competition simulation, performance enhancement.

According to another embodiment of the invention, the machine learning module comprises a long short-term memory neural network, LSTM neural network, configured to execute the first processing stage and/or at least parts of the second processing stage.

The long short-term memory neural network may be comprised by the first neural network. In case the method makes use of more than one neural network, each neural network may be a long short-term memory network.

Particularly, the LSTM neural network is a bidirectional LSTM neural network. According to a second aspect of the invention, a computer program is claimed, wherein the computer program comprises computer program code that when executed on a computer executes the method according to one of the preceding claims.

The computer program may be stored on a non-transitory storage medium.

A computer in the context of the current specification may be understood as a processor configured to execute the computer program. The computer may be comprised in a device, such as a sport watch, an ECG-system or any other device configured to acquire an input signal.

Alternatively, the computer may be a stand-alone device, particularly devoid of sensors for recording or generating the input signal, but wherein the computer is connected or connectable to or comprises a non-transitory storage medium comprising the input signal, e.g. in form of data, wherein the computer may acquire the input signal from said the storage medium, e.g. by requesting or reading said data form the storage medium.

According to a third aspect of the invention, a system is claimed, wherein the system comprises:

    a) a sensor configured to record and generate the input signal,

    b) one or more processors configured to execute the computer program according to the invention,

    c) particularly the computer program stored on a non-transitory storage medium connected to the one or more processors or the computer.

According to another embodiment of the invention, the system comprises a sensor device that comprises the sensor, a first wireless data interface, and a user interface, wherein the system further comprises a server device, wherein the server comprises the one or more processors and a second wireless data interface, wherein the first and the second data interfaces are configured to wireless exchange data comprising information on the input signal to provide the one or more processors the input signal and to exchange data comprising information on the IAT, and/or one or more selected from the list consisting of:

-    the individual load value,

-    the individual anaerobic heart rate value,

-    the individual anaerobic heart rate variability value,

-    the characteristic deflection point,

-    the individual load value associated to the characteristic deflection point,

-    the individual heart rate value associated to the characteristic deflection point,

-    the individual heart rate variability value associated to the characteristic deflection point.

This embodiment teaches a system with at least two separate entities, the sensor device, and the server.

The sensor device and the server may exchange information by way of the first and second wireless data interface. This allows transmitting the recorded input signal from the sensor of the sensor device to the processor of the server that may then determine the IAT and other parameters by way of executing the method according to the invention.

This embodiment allows executing energy-consuming operations such as hosting and executing a neural network on the server, wherein the sensor device is not required to perform these tasks, which allows implementation of the system in consumer wearable heart rate or pulse tracker devices.

The results of the method, namely at least the determined IAT, may be transmitted to the sensor device for display to the exercising subject. The sensor device may be configured to compare an instantaneous heart rate or heart rate variability that may be determined on the sensor device to the individual anaerobic heart rate value, and/or the individual anaerobic heart rate variability value, as received from the server, and to provide the subject with this information, for example by way of sensory feedback. The sensory feedback may comprise displaying the information on a display of the sensor device, providing a mechanical feedback to the subject, such as a vibration.

According to another embodiment of the invention, the system comprises a wearable heart rate or pulse tracker, such as a consumer sport watch, particularly wherein the wearable heart rate or pulse tracker is the sensor device.

This embodiment allows for implementing the method in state-of-the-art device, for improving their performance in terms of determining the IAT.

Particularly, exemplary embodiments are described below in conjunction with the Figures. The Figures are appended to the claims and are accompanied by text explaining individual features of the shown embodiments and aspects of the present invention. Each individual feature shown in the Figures and/or mentioned in said text of the Figures may be incorporated (also in an isolated fashion) into a claim relating to the device according to the present invention.

Fig. 1      schematically depicts the method according to the invention;

Fig. 2      shows a flow diagram of the method;

Fig. 3      shows the determination of individual values associated with the IAT;

Fig. 4      shows exemplary training progress applying the method; and

Fig. 5      shows an exemplary system according to the invention.

Training of the neural networks

In the following, various embodiments according to the invention are detailed exemplarily.

Depending on the specific implementation of the method and the resulting architecture of the machine learning module, differing training procedures with respect to the training data set are used.

Generally, training data sets differ for a first implementation - the following referred to as single-stage solution - in which a neural network or a neural network system is trained to execute the first processing stage, outputs the output signal, and wherein the output signal is then processed using other means than neural network or trained classifiers, but rather analytical operations yielding the IAT, as disclosed in previous paragraphs. Embodiments falling under this first implementation are referred to as two-part solution in the following paragraphs.

In contrast, a second implementation - in the following referred to as multi-stage solution - includes executing the first processing stage with a neural network, and executing operations of the second processing stage by a neural network as well. For sake of clarity, it is assumed that the neural network executing the second processing stage results in the IAT as an output of the neural network. Clearly, and as claimed, it is possible that the neural network may execute only a part of the second processing stage and thus the output would deviate from the IAT, but - for example - determine the linearized regions in the phase deflection signal. The step of determining the IAT from these linearized regions may be done outside the neural network.

The multi-stage solution may be implemented by a single neural network that processes the input signal and outputs the IAT (or an intermediate result of the second processing stage). This embodiment is referred to as the end-to-end solution Alternatively, the multi-stage solution may be implemented by two (or more) neural networks, wherein a first neural network is configured to execute the first processing stage and the second neural network is configured to execute the second processing stage; this embodiment is referred to as the split solution.

The architecture of the neural network configured for the single-stage solution and the neural network executing the first processing stage in the split solution may be identical; in fact, the neural networks may be identical.

Neural network architecture and Training for the single stage solution

Creating training data for training the machine learning module, and in particular the one or more neural networks, for the single-stage solution requires an input signal e.g. a respiratory activity signal, synchronized to an output signal, e.g. a respiratory flow signal, representative of the respiration of the subject, wherein it is important that the output signal is an unadulterated representation of the actual breathing and is not affected by interference signals.

Synchronization of these two signals may have an accuracy in the range of 50 to 2,000 milliseconds. Ideally, the two signals are recorded under laboratory conditions during an increasing load designed to lead the subject to its IAT. However, the two signals may also be generated in other environments and under different circumstances.

The two signals may then be visually inspected, and episodes with disturbances in either one of the signals should be excluded from training.

The inspected signals may be referred to as training signals. Training data for the single-stage solution comprises a plurality of training signals.

The training signal representing the respiration is assigned as the output signal. This output signal represents the ground truth for the training of the neural network, for which the neural network is trained to predict. The ground truth may be sampled at 128 Hz, for example. The ground truth may be represented as a matrix having the size of [L, 1], wherein L corresponds to the duration of the recording in seconds * sample frequency. The ground truth may be normalized to values between 0 and 1. Alternatively, or additionally, characteristic features (and their associated time points) are extracted from the training signal representative of the respiration. These features then may be considered as the output signal, which represents the ground truth to be predicted neural network. Each characteristic feature may be considered as a property of the respiratory signal. Characteristic features may include one or more of the following features

- time points of maximum inspiration during respiration of the subject,
- time points of maximum expiration during respiration of the subject,
- the start of inspiration,
- maximum inspiration flow,
- start of expiration
- maximum expiration flow.

These characteristic features alone or in combination may be considered as the output signal, and thus the ground truth.

The ground truth comprising or consisting of one or more of the characteristic features may be binary-coded in an array and sampled at, for example, with 128 Hz. The size of the array representing the ground truth may therefore be [L, N, 1], wherein L is a duration of recording in seconds * sampling frequency of the input signal. N corresponds to the number of different characteristic features included in the ground truth. The ground truth may be normalized to values between 0 and 1.

The input signal may also be sampled at, for example, 128 Hz.

The training signal, i.e. the input signal and the ground truth, are then divided into sections of equal length, e.g. 10 seconds, zero-padding at the beginning and/or end. The neural network may comprise or consist of multi-layer, bidirectional Long Short-Term Memory (LSTM) neural network. Alternatively, one or more convolutional neural networks may be employed.

In any case, the architecture has to be configured to receive the input signals from the training data. Further, the neural network is configured to output the output signal generated from the input signal.

In an advantageous embodiment, the sampling rate of the input signal and the generated output signal are identical. The size of the array representing the output signal may therefore be [S, N, 1], wherein S is a duration of the section of the output signal * sampling frequency of the output signal. N is either 1, in case the output signal corresponds to the respiratory signal, or N may correspond to the number of different characteristic features included in the output signal. The neural network may be trained according to a loss-function, that characterizes a deviation between the generated output signal and the ground truth.

Neural network architecture and Training for the split-solution

The preparation of the training signal and the training data in essence comprises all steps as laid out in the context of the single-stage solution. However, in addition to preparing the training signals, the IAT is determined to become part of the training data, as the IAT is to be determined after the executing of the second processing stage by a neural network.

The determination of the IAT may be carried out by analysis of the respiratory signal, i.e. the signal representative of the respiration as well and/or a synchronized lactate measurement recorded during the physical exercise of the subject. The physical exercise may be a stress test with a ramp or step protocol featuring an increasing exercise load.

The determined IAT is checked, e.g. by an expert and evaluated according to quality criteria. Only training signals with a clearly identifiable IATs may be used for the training.

The IAT forms a ground truth for the neural network of the second processing stage that is to be determined from the ground truth representing the output signal.

The data format of the IAT may be [IAT, 1].

In summary, the training data for the split solution comprises the training signal divided in a plurality of sections of equal length, as well as the IAT, associated to each training signal.

Training and neural network architecture for the neural network executing the first processing stage has been elaborated in the context of the single-stage solution. The neural network for the second processing stage may comprise or consist of multi-layer, bidirectional Long Short-Term Memory (LSTM) neural network. Alternatively, one or more convolutional neural networks may be employed.

The architecture of the neural network executing the second processing stage has to be configured to receive the generated output signal from the neural network that executes the first processing stage. The neural network executing the second processing stage output an IAT that is determined from the output signal of the neural network of the first processing stage. By comparing the IAT comprised in the training data with the generated IAT from the neural network allows training e.g. by way of a gradient descent method.

Neural network architecture and Training for the end-to-end-solution

According to this solution, the IAT is determined from the input signal by a single neural network. The input signal may be treated as described in the previous solutions, with the difference that the input signal is not divided into sections of equal length, but all input signals are padded to have the same length, i.e. the length of the longest input signal, e.g. 45 minutes.

The IAT as the training ground truth is determined as laid out in the previous paragraphs regarding the split solution.

The training data therefore comprises the input signals of the same length and associated IATs.

As in the previous embodiments, the neural network may be advantageously configured as a LSTM neural network. Alternatively, a convolution neural network may be used.

General Considerations regarding training and architectures of the neural network(s)

Input signals and output signals, as well as IAT, may be obtained by validation studies. For this purpose, each individual recorded signal may be personally inspected by a skilled person, e.g. assisted by automated filters. The skilled person has to mark and/or annotate any characteristic feature, e.g. time points of maximum inspiration and expiration, in the output signal after removing all intervals with insufficient measurement quality. These curated data, may be obtained from spiroergometry data and/or impedance cardiography. Thus, the output signal may be a spiroergometry signal and/or an impedance cardiography signal.

Subsequently, the input signals, e.g. including ECG, ICG, chest excursion signal are sampled to 200Hz, normalized in the range from -1 to 1 and synchronized with the curated data. The input signals and curated data are the training data. The training data may then be divided into 30 second intervals and, if necessary, with zero padding in border areas, divided into arrays with the format 2x6,000.

LSTM networks can be used to process equidistant sequence data. It is possible to use neural networks with 3, 5, 7 and 9 LSTM layers respectively, wherein the first and last layers are forward-running LSTM layers and all even rows (2, 4, 6, 8) are backward-running LSTM layers. One may vary the units per layer between 16 and 128. Complex data with a poor signal-to-noise ratio in particular benefits from more layers with more units, but these are also correspondingly slower and more memory-intensive.

The neural networks were trained using the training data. By reducing the output signal, e.g. the respiratory signal from an analog respiratory flow to a two-sided digital signal comprising characteristic features digitally coded as follows: time point of maximum inspiration = -1; time point of maximum expiration = 1; everything else = 0.

Using the characteristic features (which each represent a property of the respiratory signal), a significantly faster convergence of the training may be achieved, even with small sub-data sets. It is important to weight the outcome variables (-1; 0; 1) and not to allow any bias towards the zero values.

The trained neural networks generate an output signal either comprising the respiratory signal or one or more properties of the respiratory signal, i.e. a signal comprising one or more characteristic features of the respiratory signal. Clearly, the output signal may be generated such as to be representative of a phase of the respiratory signal, as laid out in the claims.

Figure Description

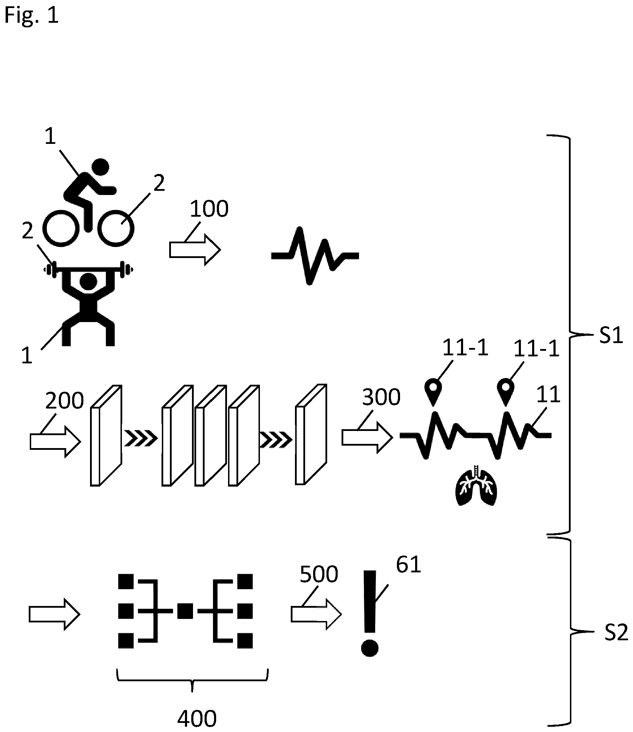

In Fig. 1 the method according to the invention is depicted schematically.

A subject 1 is exposed to a physical load 2, and exercise with increasing load. During exercise an input signal 10, here a heartbeat signal is recorded 100 and provided 200 to the machine learning module 20. The machine learning module 20 comprises a neural network 21 with a plurality of layers 21-1. The neural network 21 is configured to execute a first processing stage S1, in which from the input signal 10, an output signal 11 is generated 300. In this case, the output signal 11 comprises a respiratory signal, wherein characteristic features 11-1 are identified in the respiratory signal by the neural network 21.

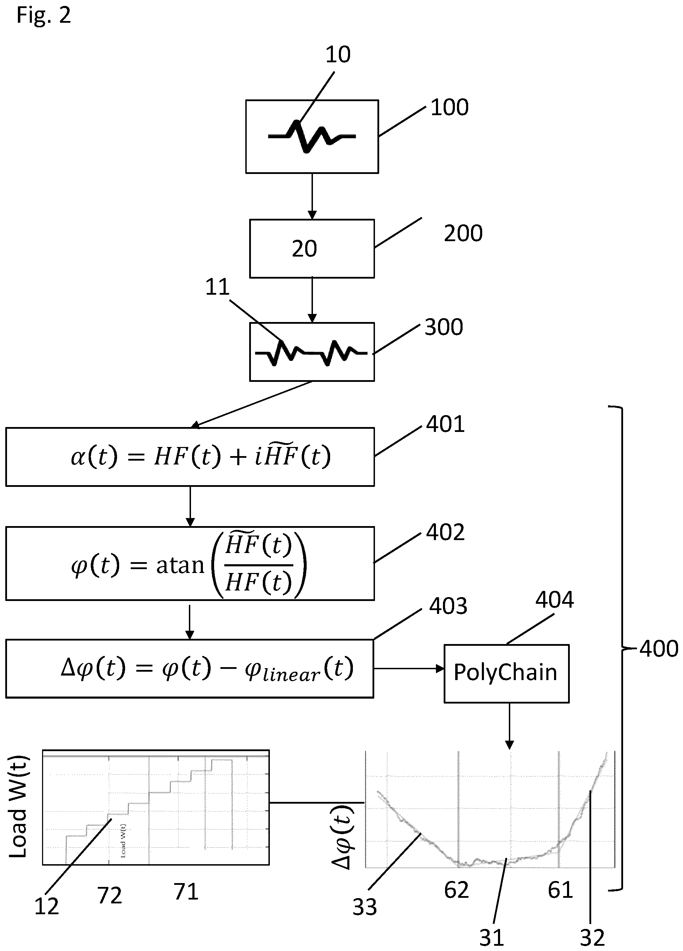

According to this example, the output signal 11 is further processed 400 by an analytical process 23 detailed in Fig. 2. From the output signal 11 the analytical processing identifies 500 the individual anaerobic threshold, IAT, 12 of the subject 1. In Fig. 2, the split-solution is elaborated in more detail.

The method comprises a first step 100 of acquiring the input signal 10. Here, the input signal 10 may be a pulse signal optically recorded by a sport watch worn at a wrist of the subject 1 or by means of a chest strap worn by the subject 1.

The input signal 10 may be continuously recorded and processed in chunks of e.g. 30 s (depending on the specific training and design of the neural network), while the subject 1 exercises under an increasing workload. The subject 1 may select the kind of work load. The workload may be an ergometric load or a rehab load. In this example, the load signal 12 increases stepwise and is recorded synchronous to the input signal 10.

The neural network 21 processes 200 the input signal 10, generates 300 and puts out the output signal 11, in this case the respiratory signal.

In a next step 401, the respiratory signal $HF(t)$ as determined by the neural network 21 is transformed by means of a Hilbert Transform. From the Hilbert transformed signal, an analytical signal $\alpha(t)$ may be determined:

$$\alpha(t) = HF(t) + i\widetilde{HF}(t),$$

wherein $\widetilde{HF}(t)$ is the Hilbert conjugate of $HF(t)$. From the Hilbert conjugate $\widetilde{HF}(t)$ and the filtered signal $HF(t)$, a phase signal <p(t) according to $\varphi(t) = \text{atan}\left(\frac{\widetilde{HF}(t)}{HF(t)}\right)$, atan is determined 402.

From the phase signal <p(t), the phase deflection signal 13 is determined 403. For this, the phase signal is decomposed in a linear phase signal $\varphi_{linear}(t)$, representing a linear phase contribution of the phase signal, wherein the phase deflection signal 13 is the difference between the phase signal and the linear phase contribution:

$$\Delta\varphi(t) = \varphi(t) - \varphi_{linear}(t)$$

From the phase deflection signal 13 $\Delta\varphi(t)$ three linearized regions - a first 31, a second 32, and a third linearized region 33, are determined, e.g. by fitting a polygonal chain with three linear segments 41, 42, 43 connected by two vertices 51, 52. The phase deflection signal 13 is in essence U-shaped, wherein the first linearized region 31 is delimited between the third linearized region 33 in which a linearized slope of the phase deflection signal 13 is negative and the second linearized region 32 in which a slope of the linearized phase deflection signal 13 is positive and greater than a linearized slope of the phase deflection signal 13 of the first linearized region 31.

The vertex 52 connecting the segments associated to the third linearized region 33 and the first linearized region 31 may be associated to a characteristic deflection point 62, which indicates a transition from a resting phase or warm-up phase with mixed contribution of anaerobic and aerobic metabolic activity to an exclusively aerobic regime of the metabolic activity.

The vertex 51 connecting the segments 41, 42 associated to the first linearized region 31 and the second linearized region 32 is associated to the individual anaerobic threshold 61, a time point in which a respiratory exchange ratio (RER) is in the range of 0.88 to 0.92. The RER indicates the fraction of carbon dioxide production and oxygen consumption of the subject. It is a time point during the exercise at which the cell metabolism switches from an aerobic to anaerobic metabolism.

As the load signal 12 is recorded synchronous to the input signal 10 and thus the output signal 11, an individual anaerobic load value 71 in the load signal 12 may be determined, wherein said load value 71 corresponds to the load at the time of the IAT 61 in the load signal 12. Similarly, an individual anaerobic heart rate value 81 or heart rate variability value may be determined by assigning the time of the IAT 61 to the corresponding heart rate signal 14 / heart rate variability signal. Clearly, an individual anaerobic pulse rate may be assigned to the IAT in case a synchronous pulse signal is available.

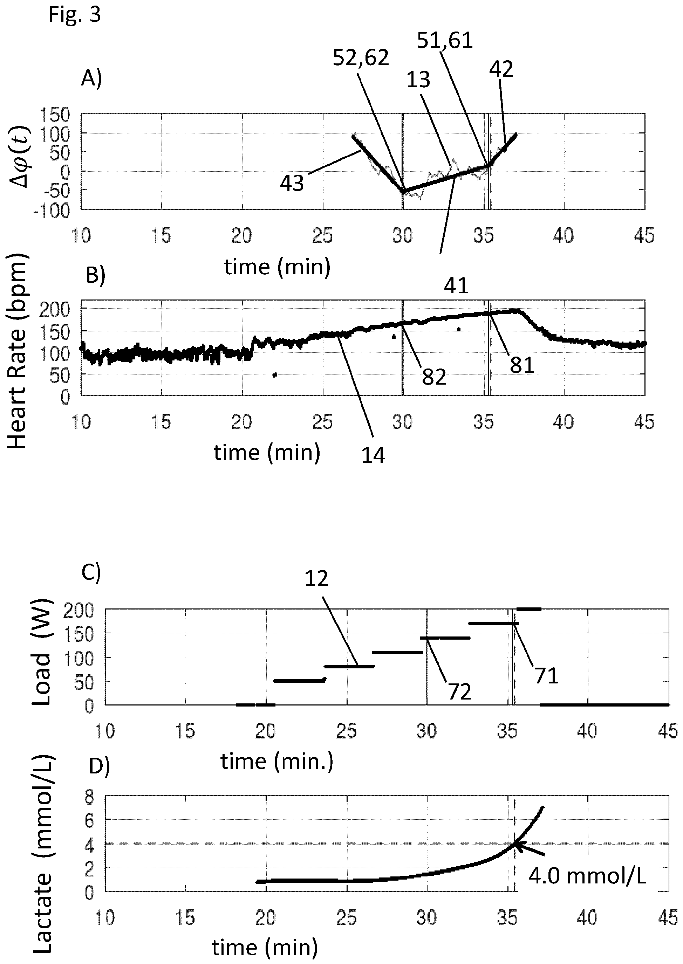

Fig. 3 shows the corresponding signals 13, 12, 14, with IAT 61 and the associated individual heart rate value 81, the individual load value 71.

In Panel A, the phase deflection signal 13 is shown with the linearized regions 31,32,33 as well as the determined characteristic deflection point 62 and the IAT 61. In Panel B, the heart rate signal 14 is shown, with the corresponding the individual anaerobic heart rate value 81.

In Panel C the load signal 12 is shown, indicating the individual anaerobic load value 71.

In Panel D, the lactate level has been determined from the subject 1. At the IAT the subject exhibits a lactate level of 4.0 mmol/L.

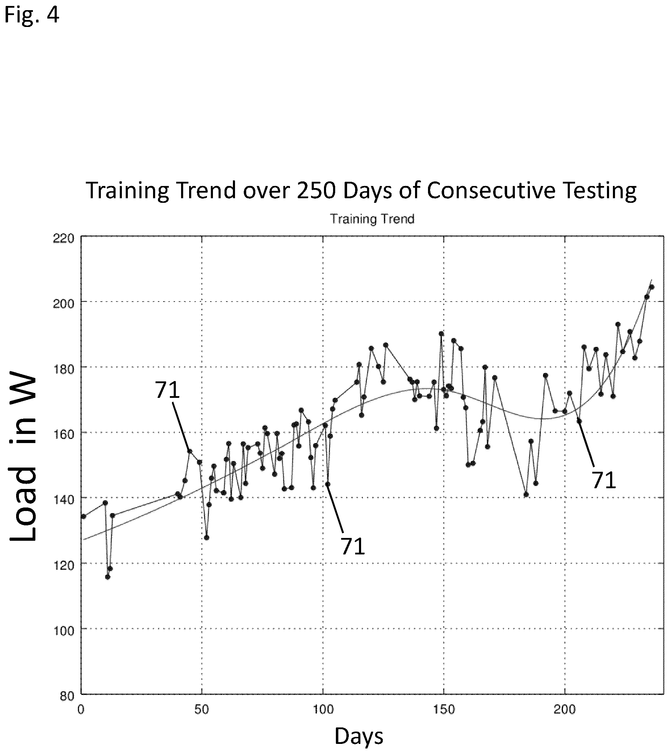

These results may be used to monitor or improve physical training of the subject 1. This is shown in Fig. 4. In Fig. 4 the individual anaerobic load value 71 has been determined over the course of more than 200 days (each measurement is depicted as a circle connected by a straight line, wherein a moving average line is drawn to remove the noise), wherein the subject 1 worked out at its IAT over the course of the experiment. For this, the subjects' heart rate signal 14 was monitored, and the IAT was determined by processing the heart rate signal 14 according to the method of the invention. The subject was instructed to aim its workout intensity at the individual IAT e.g. in a range of -10% to +10% of the IAT. As can be seen, the effect of this training, the individual anaerobic load value 71 increases from about 128 W to about 200 W.

The method according to the invention may be used to train in personalized training zones:

> As laid in preceding sections of the specification, the IAT can be used to establish individualized training zones, by e.g. by defining a range for the heart rate or the heart rate variability relative to the IAT. Training in a range just below or at the IAT ensures that the exercise intensity remains sustainable and predominantly aerobic, reducing fatigue and avoiding excessive lactate accumulation. This is particularly advantageous for endurance athletes aiming to improve performance or for patients with limited cardiovascular reserve.

Determination of the onset of a stable metabolic phase, corresponding to the characteristic deflection point, on the other hand, helps to ensure a balance between lactate production and clearance during training. Training in a range above the characteristic deflection point (or its associated heart rate or heart rate variability) can enhance both aerobic and anaerobic capacity over time.

The method may also be used for progressive overload training:

By identifying the IAT, trainers or therapists can implement a gradual increase in training intensity over the period of days and weeks to improve the body's ability to tolerate higher workloads. This is especially useful for athletes and recovering patients seeking to improve endurance or return to normal function after injury or illness.

For the progressive overload training, training in the range at and slightly above the IAT is desirable, e.g. 5% to 15% above the IAT.

Further, the method may be used for fatigue management and recovery:

The onset of the stable metabolic phase (at the characteristic deflection point) provides a precise intensity level at which the body is operating at its maximum steady-state metabolic efficiency. Training at or just below the characteristic deflection point, or at its associated heart rate or heart rate variability, can prevent overtraining while stimulating improvements in aerobic capacity, making it ideal for recovery sessions or rehabilitation protocols.

The method may further be advantageously applied in cardiovascular disease management:

For patients with cardiovascular conditions, training below the IAT ensures a safe exercise intensity, promoting cardiovascular health without overstraining the heart. This is critical for improving oxygen uptake, reducing blood pressure, and enhancing circulation without exacerbating the condition.

The onset of a stable metabolic phase can be used to define a safe upper limit for exercise, ensuring that the patient maintains an intensity level that promotes metabolic balance and avoids the onset of ischemic symptoms or arrhythmias.

The method may be used to improve metabolic efficiency:

Training at or near the onset of a stable metabolic phase, i.e. the characteristic deflection point or its associated heart rate value or heart rate variability value, can help improve mitochondrial efficiency and the ability to utilize fats and carbohydrates as energy sources. This is particularly beneficial for individuals with metabolic disorders, diabetes, or obesity.

The method may be used in post-surgical or chronic disease recovery:

After surgical procedures such as bypass surgery or valve repair, training below the IAT allows gradual cardiovascular adaptation without excessive stress. Regular monitoring of the IAT and onset of a stable metabolic phase ensures progression while minimizing the risk of setbacks. For patients with chronic conditions, maintaining exercise within the onset of a stable metabolic phase range provides a controlled way to improve functional capacity over time, helping them regain independence and improve quality of life.

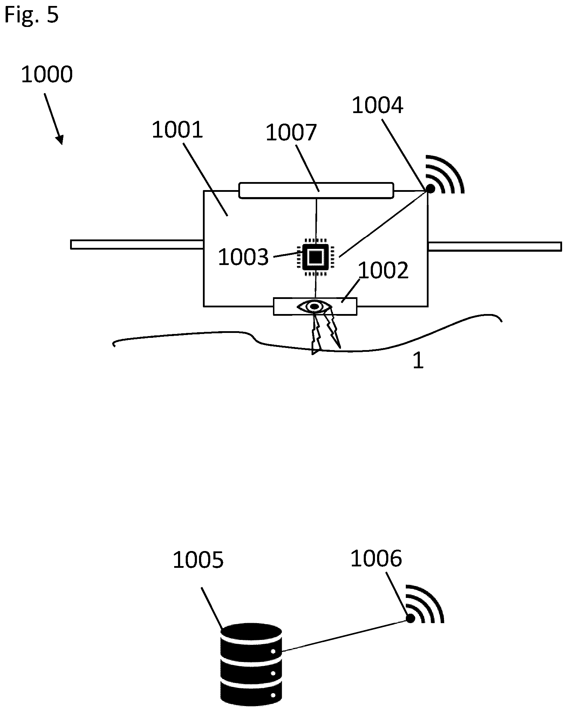

In Fig. 5 a system 1000 according to the invention is schematically shown. The system 1000 comprises a wearable heart rate tracker 1001, such as a smartwatch, or a chest strap. The wearable heart rate tracker 1001 comprises an optical sensor 1002 configured to record a pulse or heart rate signal from the subject 1 wearing the wearable heart rate tracker 1001. The sensor 1002 is connected to a processor 1003 that is configured to execute at least part of the method. The processor 1003 may for example be configured to filter or process the input signal 10, i.e. the signal recorded from the sensor 1002.

The processing of the input signal 10 may comprise execution of the first and the second processing stage S1, S2.

Alternatively, the processing may comprise solely some pre-processing of the sensor signal to arrive at the input signal 10. The processor 1003 is connected to a first data interface 1004 that is configured to wireless connect the processor 1003 with a server 1005 that is connected to a second data interface 1006.

According to one embodiment, the processor 1003 sends the pre-processed sensor signal as input signal 10 via the data interfaces 1004, 1006 to the server 1005, wherein the server 1005 executes the first and/or the second processing stage S1, S2. The results of the processing at the server 1005 may be sent back to the wearable heart rate tracker 1001. There, any missing processing steps, e.g. the second stage processing S1 may be executed (in case it has not been executed by the server 1005 already). The result of the second processing stage S2 may be displayed on a display 1007 of the wearable heart rate tracker 1001. For example, it may be that the wearable heart rate tracker 1001 indicates to a subject 1, whether a heart rate is within the individual anaerobic heart rate value, such as to control a training intensity.

The wearable heart rate tracker 1001 may also store these results from the processing of the second processing stage S2 and may further provide a temporal course of these results, e.g. displaying a history of the individual anaerobic heart rate values, and/or individual anaerobic load values.

Reference numerals

| 1 | subject |
| 2 | physical load |
| 10 | input signal |
| 11 | output signal |
| 12 | work load signal |
| 13 | phase deflection signal |
| 14 | heart rate signal |
| 11-1 | characteristic feature of output signal |
| 20 | machine learning module |
| 21 | neural network |
| 21-1 | layer of neural network |
| 31 | first linearized region |
| 32 | second linearized region |
| 33 | third linearized region |
| 41,42,43 | linear chain segments |
| 51 | first vertex |
| 52 | second vertex |
| 61 | IAT |
| 62 | characteristic deflection point |
| 71 | individual anaerobic load value |
| 72 | load value at characteristic deflection point |
| 81 | individual anaerobic heart rate value |
| 72 | heart rate value at characteristic deflection point |
| 100 | acquiring the input signal |
| 200 | transmitting input signal to the machine learning module |
| 300 | generating the output signal |
| 400 | processing the output signal |
| 401 | determining an analytical Hilbert transformed signal |

| 402 | determining a phase conjugate signal |
| 403 | determining the phase deflection signal |
| 404 | fitting a polychain to phase deflection signal |
| 500 | determining the IAT |
| 1000 | system |
| 1001 | heart rate tracker |
| 1002 | sensor |
| 1003 | processor |
| 1004 | first data interface |
| 1005 | server |
| 1006 | second data interface |
| 1007 | display/user interface/touch screen |
| S1 | first processing stage |
| S2 | second processing stage |

**Claims**

1. A method for determining an individual anaerobic threshold (61), the method comprising the steps of:

a) Acquiring an input signal (10), comprising a respiratory activity signal of a subject (1) executing a physical exercise,
b) Processing said input signal (10), comprising

• A first processing stage (S1) during which an output signal (11) is determined (300) from the input signal (10), wherein the output signal (11) is indicative of a respiration and/or of a property of the respiration of the subject (1),
• A second processing stage (S2) during which the individual anaerobic threshold (61) is determined from the output signal (11), the individual anaerobic threshold (61) being a time point during the physical exercise, or an associated value at this time point, **characterized in that**

the processing of the input signal (10) comprises submitting (200) the input signal (10) to a machine learning module (20),
wherein the machine learning module (20) comprises one or more trained neural networks (21), that execute the first and/or at least parts of the second processing stage (S1, S2).

2. The method according to claim 1, wherein the output signal (11) is one or more of the list consisting of:

- a respiratory signal representing the respiration of the subject (1),
- a phase signal of the respiratory signal representing the respiration of the subject, wherein the phase signal represents an instantaneous phase of the respiratory signal,
- a phase deflection signal (13) representative of a deviation of an instantaneous phase of the respiratory signal from a linear phase of the respiratory signal,
- an inspiration signal representing time points of maximum inspiration during respiration of the subject,
- an expiration signal representing time points of maximum expiration during respiration of the subject,
- a respiration signal representing time multiple time points along each respiration cycle in the respiratory signal.

3. The method according to claim 1 or 2, wherein the machine learning module (20) consists of one trained neural network (21) that executes the first and at least parts of the second processing stage, particularly the complete second processing stage.

4. The method according to claim 1 or 2, wherein the machine learning module comprises a first trained neural network (21) that executes the first processing stage.

5. The method according to one of the preceding claims, wherein the machine learning module comprises a second trained neural network that executes at least a part of the second processing stage or the complete second processing stage.

6. The method according to claims 4 and 5, wherein the first and the second neural network are configured to execute all steps of the first and the second processing stage (S1, S2).

7. The method according to one of the preceding claims, wherein from the output signal (11), in case it is not the phase deflection signal (13), the phase deflection signal (13) is determined, wherein from the phase deflection signal (1§) the individual anaerobic threshold (61) is determined.

8. The method according to claim 7, wherein determining the individual anaerobic threshold (61) comprises determining a first and a second linearized region (31, 32) in the phase deflection signal (13), wherein the first linearized region (31) precedes the second linearized region (32) and is **characterized by** a slope with a first slope value, wherein the second linearized region (32) is located adjacent to the first linearized region (31) and exhibits a positive slope with a second slope value, wherein the second slope value is greater than the first slope value, wherein the individual anaerobic threshold (61) is assigned to a location of an interface of the first and the second linearized region (31, 32).

9. The method according to one of the preceding claims, wherein a load signal (12) indicative of a physical load is acquired, wherein an individual anaerobic load value (71) is determined, wherein the individual anaerobic load value (71) corresponds to the physical load at the individual anaerobic threshold (61).

10. The method according to one of the preceding claims, wherein a heart rate signal (14) is acquired, wherein an individual anaerobic heart rate value (81) is determined from the heart rate signal (14), wherein the individual anaerobic heart rate value (81) corresponds to the heart rate at the location of the individual anaerobic threshold (61), and/or wherein an individual anaerobic heart rate variability value is determined from the heart rate signal (14), wherein the individual anaerobic heart rate variability value corresponds to a heart rate variability at the position of the individual anaerobic threshold (61).

11. The method according to one of the preceding claims, wherein the input signal (10) is selected from one or more of the group consisting of:

d) an ECG signal,
e) a heart rate signal (14),
f) an electrical signal comprising the heart rhythm, such as an impedance cardiography signal,
g) a breathing signal,
h) a thorax excursion signal,
i) a pulse wave signal indicative of a pulse of the subject.

12. The method according to one of the preceding claims, wherein after the individual anaerobic heart rate value (81) and/or individual anaerobic heart rate variability value has been determined, a training method is executed, wherein the training method comprises determining the pulse or heart rate signal (14) with a wearable heart rate tracker (1001) configured to determine a heart rate signal (14), wherein the training method adjusts an exercise load such that the exercising subject's heart rate is within a range relative to the anaerobic heart rate value (81) and/or the individual anaerobic heart rate variability value, or wherein the training method instructs the exercising subject to adjust a workout intensity such that the heart rate is within the range.

13. A computer program comprising computer program code that when executed on a computer executes the method according to one of the preceding claims.

14. A system (1000) comprising

    a) a sensor (1002)configured to record the input signal (10),
    b) one or more processors (1003) configured to execute the computer program of claim 13

15. The system(1000) according to claim 15, wherein the system (1000)comprises a sensor device (1001) that comprises the sensor (1002), a first wireless data interface (1004), and a user interface (1007), wherein the system (1000) further comprises a server (1005), wherein the server (1005) comprises the one or more processors and a second wireless data interface (1006), wherein the first and the second data interfaces (1004,1006) are configured to wireless exchange data comprising information on the input signal (10) to provide the one or more processors the input signal (10) and to exchange data comprising information on the individual anaerobic threshold (61), and/or one or more selected from the list consisting of:

    - the individual anaerobic load value (71),
    - the individual anaerobic heart rate value (81),
    - the individual anaerobic heart rate variability value,
    - the characteristic deflection point (62),
    - the individual load value (72) associated to the characteristic deflection point (62),
    - the individual heart rate value (82) associated to the characteristic deflection point (62),
    - the individual heart rate variability value associated to the characteristic deflection point (62).

Fig. 1

Fig. 2

$$\alpha(t) = HF(t) + i\widetilde{HF}(t)$$

$$\varphi(t) = \text{atan}\left(\frac{\widetilde{HF}(t)}{HF(t)}\right)$$

$$\Delta\varphi(t) = \varphi(t) - \varphi_{linear}(t)$$

PolyChain

Fig. 3

Fig. 4

Training Trend over 250 Days of Consecutive Testing

Fig. 5

1000

1001   1007   1004

1003   1002

1

1005   1006

# EUROPEAN SEARCH REPORT

**Application Number**

EP 24 22 2789

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/029997 A2 (NEUMAFIT CO LTD [KR]) 8 February 2024 (2024-02-08) * the whole document * | 1-15 | INV. A61B5/08 A61B5/22 A61B5/00 |
| X | US 2023/033967 A1 (RICHARDS DYLAN [US]) 2 February 2023 (2023-02-02) * paragraphs [0006], [0029], [0035], [0057], [0077] - [0107], [0115] - [0155], [0167] - [0171]; claims; figures * | 1-15 | ADD. A61B5/113 A63B24/00 |
| X | WO 2018/049531 A1 (OMSIGNAL INC [CA]) 22 March 2018 (2018-03-22) * paragraphs [0003] - [0007], [0028] - [0032], [0049] - [0081], [0107] - [0110]; claims; figures * | 1-15 | |
| X | WO 2024/105592 A1 (DYSON TECHNOLOGY LTD [GB]) 23 May 2024 (2024-05-23) * page 1, line 27 - page 2, line 8 * * page 5 - page 9; claims; figures * | 1,2,4, 7-15 | |
| A | OLEG ANOSOV ET AL: "High-frequency oscillations of the heart rate during ramp load reflect the human anaerobic threshold", EUROPEAN JOURNAL OF APPLIED PHYSIOLOGY, SPRINGER VERLAG, HEIDELBERG, DE, vol. 83, no. 4-5, 1 November 2000 (2000-11-01), pages 388-394, XP002626426, ISSN: 1439-6319, DOI: 10.1007/S004210000302 * the whole document * | 1-15 | **TECHNICAL FIELDS SEARCHED (IPC)** A61B A63B |

The present search report has been drawn up for all claims

| Place of search | Date of completion of the search | Examiner |
|---|---|---|
| The Hague | 15 May 2025 | Crisan, Carmen-Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 22 2789

This annex lists the patent family members relating to the patent documents cited in the above-mentioned European search report.
The members are as contained in the European Patent Office EDP file on
The European Patent Office is in no way liable for these particulars which are merely given for the purpose of information.

15-05-2025

| Patent document cited in search report | Publication date | Patent family member(s) | Publication date |
|---|---|---|---|
| WO 2024029997 A2 | 08-02-2024 | NONE | |
| US 2023033967 A1 | 02-02-2023 | CN 114616628 A | 10-06-2022 |
| | | EP 4000074 A1 | 25-05-2022 |
| | | US 2021020293 A1 | 21-01-2021 |
| | | US 2023033967 A1 | 02-02-2023 |
| | | US 2024347164 A1 | 17-10-2024 |
| | | WO 2021011381 A1 | 21-01-2021 |
| WO 2018049531 A1 | 22-03-2018 | CA 3046375 A1 | 22-03-2018 |
| | | EP 3515301 A1 | 31-07-2019 |
| | | WO 2018049531 A1 | 22-03-2018 |
| WO 2024105592 A1 | 23-05-2024 | GB 2624434 A | 22-05-2024 |
| | | WO 2024105592 A1 | 23-05-2024 |

EPO FORM P0459

For more details about this annex : see Official Journal of the European Patent Office, No. 12/82